# EUROPÄISCHE PATENTSCHRIFT

(11) **EP 0 403 947 B1**
(45) Veröffentlichungstag und Bekanntmachung des Hinweises auf die Patenterteilung: **20.07.1994**
(21) Anmeldenummer: 90111175.7
(22) Anmeldetag: 13.06.1990
(51) Int. Cl.: C07C 309/86, C07C 309/88, C07C 309/89

(54) **Verfahren zur Herstellung von aromatischen Sulfonsäurechloriden**
Process for the preparation of aromatic sulfonic acid chlorides
Procédé pour la préparation de chlorure d'acide sulfonique aromatique

(30) Priorität: 17.06.1989 DE 3919840
(43) Veröffentlichungstag der Anmeldung: 27.12.1990
(73) Patentinhaber: HOECHST AKTIENGESELLSCHAFT, 65926 Frankfurt am Main (DE)
(72) Erfinder: Meier, Michael, Dr., D-6000 Frankfurt am Main (DE); Tronich, Wolfgang, Dr., D-6239 Eppstein (DE)

(56) Entgegenhaltungen:
- EP-A- 0 001 275
- DE-A- 3 302 647
- DE-A- 3 306 597
- DE-A- 3 501 754
- HELVETICA CHIMICA ACTA. vol. 42, no. 176, 1959, BASEL CH Seiten 1653 - 1658; H.H. BOSSHARD et al.: "Eine Methode zur Katalysierten Herstellung von Carbonsäure- und Sulfosäure-Chloriden mit Thionylchlorid"
- OTTO LINDNER: "ULLMANN'S ENCYCLOPEDIA OF INDUSTRIAL CHEMISTRY" no. A3, 1985,VCH, WEINHEIM
- CHEMISCHE TECHNOLOGIE vol. 6, 1982, CARL-HAUSER-VERLAG MÜNCHEN WIEN WINNACKER-KÜCHLER: "3 HERSTELLUNG VON MONOCYCLISCHEN AROMATEN"

## Beschreibung

Die Erfindung betrifft die Herstellung von aromatischen Sulfonsäurechloriden in an sich bekannter Weise durch Umsetzung von aromatischen Verbindungen mit überschüssiger Chlorsulfonsäure oder mit Chlorsulfonsäure bzw. Oleum und Thionylchlorid, wobei in Gegenwart von Amidosulfonsäure als Katalysator gearbeitet wird.

Es ist bekannt, daß die Sulfonierung und Sulfochlorierung von Aromaten verbessert werden kann, indem zur Unterdrückung der als Nebenreaktion beobachteten Sulfonbildung sogenannte Sulfoninhibitoren zugesetzt werden [Ullmann's Encyclopedia of Industrial Chemistry, 5. Aufl., Vol. A.3, S. 513]. Als Beispiele für solche Sulfoninhibitoren seien Ammoniumsulfat, Natrium- oder Kaliumcarbonat [DE 35 01 754], sowie Natrium- oder Ammoniumchlorid [DE 33 06 597] genannt. Eine Vielzahl weiterer Sulfonierungshilfsmittel wird in der EP 0 001 275 erwähnt.

Aus Helv. Chimica Acta 42, 1653 (1959) ist bekannt, daß Dialkylformamide als Katalysatoren bei der Herstellung von Naphthalinsulfonsäurechloriden aus den entsprechenden Sulfonsäuren wirken.

In DE 29 28 744 wird auf die toxikologischen Probleme bei der Verwendung von insbesondere Dimethylformamid hingewiesen, und es werden Ersatzstoffe, wie beispielsweise Pyridin, substituierte Pyridine, tertiäre aliphatische Amine und quartäre Ammoniumsalze, bevorzugt tertiäre Amine, vorgeschlagen.

In der DE 3 302 647 wird ein Verfahren zur Herstellung von 4-Chlorphenylsulfonylchlorid durch Umsetzung von Chlorbenzol mit Chlorsulfonsäure und Thionylchlorid in Anwesenheit von Dialkylamiden, Tetramethylharnstoff oder Triethylamin als Katalysatoren beschrieben.

Die für die Sulfonierung bzw. Sulfochlorierung von Aromaten als Katalysatoren oder Hilfsmittel vorgeschlagenen Verbindungen sind für eine industrielle Verwendung, insbesondere aus gewerbehygienischen Gründen, nicht befriedigend und somit verbesserungsfähig.

Es wurde nun gefunden, daß Amidosulfonsäure ein neues, vorteilhaftes Hilfsmittel für die Sulfochlorierung von Aromaten zu den entsprechenden Arylsulfonsäurechloriden ist.

Gegenstand der Erfindung ist somit ein hinsichtlich erzielbarer Ausbeute und Qualität verbessertes Verfahren zur Herstellung aromatischer Sulfonsäurechloride der allgemeinen Formel I 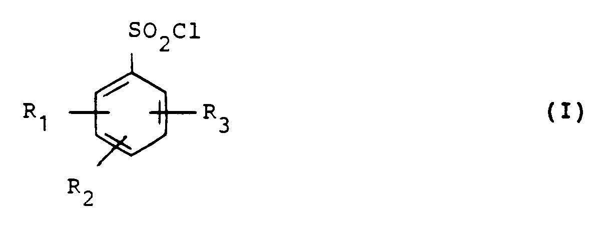
in welcher R₁, R₂ und R₃ gleich oder verschieden sind und Wasserstoff-, Fluor-, Chlor-, Brom- oder Jodatome, Alkyl(C₁-C₄)-, Acetylamino-, Nitro- oder Carboxylgruppen bedeuten, oder R₁ und R₂ gemeinsam einen aromatischen oder heteroaromatischen Ring mit 5 oder 6 Ringgliedern bilden, welcher durch Fluor-, Chlor-, Brom- oder Jodatome, Alkyl(C₁-C₄)-, Acetylamino-, Nitro- oder Carboxylgruppen substituiert sein kann, durch Umsetzung von Aromaten der allgemeinen Formel II 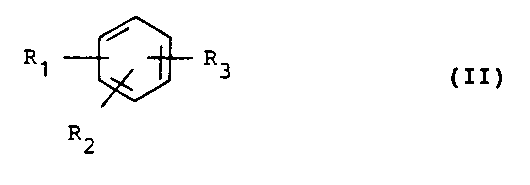
in welcher R₁, R₂ und R₃ die vorstehend genannten Bedeutungen haben, in an sich bekannter Weise mit Chlorsulfonsäure im Überschuß oder mit Chlorsulfonsäure bzw. Oleum und Thionylchlorid, dadurch gekennzeichnet, daß man in Gegenwart von Amidosulfonsäure als Katalysator umsetzt.

Die erfindungsgemäß als katalytisch wirksames Hilfsmittel eingesetzte Amidosulfonsäure läßt sich in keine der bekannten Verbindungsklassen von Hilfsmitteln bzw. Katalysatoren für die Sulfochlorierung von Aromaten einordnen.

Die Herstellung der aromatischen Sulfonsäurechloride aus den Aromaten erfolgt nach an sich bekannten Verfahren, beispielsweise durch Umsetzung der Aromaten der vorstehend genannten allgemeinen Formel II mit überschüssiger Chlorsulfonsäure [vgl. Winnacker-Küchler, Chemische Technologie, Band 6, 4. Aufl., Seite 181, Carl-Hauser-Verlag München Wien] bzw. Chlorsulfonsäure (bzw. Oleum) und Thionylchlorid [DE 33 02 647].

Hierbei verfährt man zweckmäßigerweise so, daß die Amidosulfonsäure teilweise oder vollständig der Chlorsulfonsäure zugesetzt und bei einer nur teilweisen Zugabe zu Beginn der Reaktion weitere Amidosulfonsäure während der Umsetzung zudosiert wird.

Es ist jedoch auch möglich, die aromatische Ausgangsverbindung zuerst mit Chlorsulfonsäure oder Oleum in die aromatische Sulfonsäure zu überführen, zu dem hierbei angefallenen Reaktionsgemisch die Amidosulfonsäure zuzugeben und anschließend mit Thionylchlorid weiter zum Arylsulfonsäurechlorid umzusetzen.

Die Amidosulfonsäure wird zweckmäßigerweise in einer Menge von etwa 0,1 bis etwa 20 Gewichtsprozent, vorzugsweise etwa 0,5 bis etwa 5 Gewichtsprozent, bezogen auf den Aromat der genannten allgemeinen Formel (II), der im Überschuß angewandten Chlorsulfonsäure (beim Arbeiten ohne Thionylchlorid) bzw. nach Beendigung der Sulfonierung in erster Stufe und vor Zusatz des Thionylchlorids in zweiter Stufe zugegeben. Ein Zusatz von Amidosulfonsäure in größeren Mengen ist zwar möglich, bringt aber keinerlei Vorteile mit sich.

Was die Temperaturen anbelangt, so können die bei der bekannten Sulfochlorierung aromatischer Verbindungen mit einem Überschuß von Chlorsulfonsäure, d.h. Temperaturen von etwa -10°C bis etwa 150°C, vorzugsweise von etwa 20°C bis etwa 120°C, bzw. die bei der bekannten Sulfonierung von Aromaten mit Chlorsulfonsäure bzw. Oleum in erster Stufe, d.h. Temperaturen von etwa -10°C bis etwa 150°C, vorzugsweise von etwa 20°C bis etwa 130°C, und die bei der sich anschließenden bekannten Sulfochlorierung mit Thionylchlorid in zweiter Stufe, d.h. Temperaturen von etwa 30°C bis etwa 150°C, vorzugsweise etwa 50°C bis etwa 130°C, angewandt werden.

Die nachstehenden Beispiele und Vergleichsbeispiele dienen zur Erläuterung des erfindungsgemäßen Verfahrens ohne es darauf zu beschränken.

### Beispiel 1 (4-Chlorbenzolsulfonsäurechlorid)

Zu 122 g (1,05 mol) Chlorsulfonsäure und 1 g Amidosulfonsäure werden bei 70°C über einen Zeitraum von 1 Stunde 112,6 g (1,0 mol) Chlorbenzol zugetropft. Dann wird 15 Minuten bei dieser Temperatur nachgerührt.

Anschließend werden bei 70°C 180g (1,5 mol) Thionylchlorid über einen Zeitraum von 2 Stunden zugetropft und bis zum Ende der Gasentwicklung nachgerührt. Das überschüssige Thionylchlorid wird abdestilliert und der Rückstand langsam in Eiswasser gegeben. Das ausgefallene 4-Chlorbenzolsulfonsäurechlorid wird abgesaugt und mit Eiswasser gewaschen. Es werden 202,3 g feuchtes 4-Chlorbenzolsulfonsäurechlorid mit einem Wassergehalt von 4 % erhalten. Dies entspricht 194,2 g 4-Chlorbenzolsulfonsäurechlorid (92 % d.Th.), trocken gerechnet mit einem Reingehalt von 95 %.

### Beispiel 2 (4-Chlorbenzolsulfonsäurechlorid)

Zu 122 g (1,05 mol) Chlorsulfonsäure werden bei 70°C über einen Zeitraum von 1 Stunde 112,6 g (1,0 mol) Chlorbenzol zugetropft. Dann wird 15 Minuten bei dieser Temperatur nachgerührt. Anschließend wird 1 g Amidosulfonsäure zugesetzt und bei 70°C 180 g (1,5 mol) Thionylchlorid innerhalb von 2 Stunden zugetropft. Die Aufarbeitung erfolgt wie in Beispiel 1 beschrieben. Es werden 228,3 g feuchtes 4-Chlorbenzolsulfonsäurechlorid mit einem Wassergehalt von 12 % erhalten. Dies entspricht 200,9 g 4-Chlorbenzolsulfonsäurechlorid (94,9 % d.Th.), trocken gerechnet mit einem Reingehalt von 95 %.

### Beispiel 3 (4-Chlorbenzolsulfonsäurechlorid); Vergleichsbeispiel ohne Verwendung von Amidosulfonsäure

122 g (1,05 mol) Chlorsulfonsäure, 112,6 g (1,0 mol) Chlorbenzol und 180 g (1,5 mol) Thionylchlorid werden, wie in Beispiel 1 beschrieben, jedoch ohne Zusatz von Amidosulfonsäure, umgesetzt.

Es werden 172,8 g feuchtes 4-Chlorbenzolsulfonsäurechlorid mit einem Wassergehalt von 7,1 % erhalten. Dies entspricht 160,5 g 4-Chlorbenzolsulfonsäurechlorid (76 % d.Th.) mit einer Reinheit von 94 %.

### Beispiele 4 bis 11

Zu 1 mol Chlorsulfonsäure wird 1 mol eines Aromats der nachstehenden Tabelle 1 bei 70°C zugetropft. Anschließend wird 15 Minuten nachgerührt. Dann wird zuerst, wie in Tabelle 1 angegeben, mit 1 g Amidosulfonsäure versetzt, daraufhin 2 mol Thionylchlorid zugetropft und schließlich bis zum Ende der Gasentwicklung nachgerührt. Zur Aufarbeitung wird destilliert.

Zum Vergleich wird auch die Reaktion ohne Zusatz von Amidosulfonsäure durchgeführt.

**Tabelle 1**

| **Bsp. Nr.** | **Aromat** | **Katalysator** | **Sdp.** | **Ausbeute (% d.Th.)** | **Reinheit (GC- Flächen-%)** |
|---|---|---|---|---|---|
| 4 | Benzol | mit | 142°C/40 Torr | 88,9 | 98,3 |
| 5 | Benzol | ohne | 142°C/40 Torr | 78,6 | 98,3 |
| 6 | Fluorbenzol | mit | 124°C/ 8 Torr | 84,8 | 97,0 |
| 7 | Fluorbenzol | ohne | 124°C/ 8 Torr | 76,0 | 96,9 |
| 8 | Toluol | mit | 110°C/ 2 Torr | 89,9 | 76 p- |
| | | | | | 24 o- |
| 9 | Toluol | ohne | 110°C/ 2 Torr | 80,3 | 76 p- |
| | | | | | 24 o- |
| 10 | Cumol | mit | 136°C/ 2 Torr | 85,8 | 96,7 |
| 11 | Cumol | ohne | 136°C/ 2 Torr | 67,5 | 94,0 |

### Beispiel 12 (2-Nitrotoluol- 4-sulfonsäurechlorid)

Zu 535,9 g (4,6 mol) Chlorsulfonsäure und 2 g Amidosulfonsäure werden 137,1 g (1,0 mol) o-Nitrotoluol so zugetropft, daß die Temperatur 40°C nicht überschreitet. Anschließend wird bei 40°C 1 Stunde nachgerührt und langsam auf 105°C aufgeheizt. Anschließend wird 6 Stunden bei 105°C nachgerührt, abgekühlt und bei 0 bis 5°C in Eiswasser eingetropft. Die ausgefallenen Kristalle werden abgesaugt und mit Eiswasser gewaschen. Es werden 236,1 g 2-Nitrotoluol-4-sulfonsäurechlorid (Wassergehalt 10,8 %), entsprechend 210,6 g (89 % d.Th.) mit einem Schmelzpunkt von 40°C erhalten.

### Beispiel 13 (2-Nitrotoluol-4-sulfonsäurechlorid) - Vergleichsbeispiel -

535,9 g (4,6 mol) Chlorsulfonsäure werden mit 137,1 g (1,0 mol) o-Nitrotoluol analog Beispiel 12, jedoch ohne Amidosulfonsäure, umgesetzt. Ausbeute 200,7 g (Wassergehalt 6,3 %), entsprechend 188,1 g (80 % d.Th.).

### Beispiel 14 (4-Acetamidobenzolsulfonsäurechlorid)

Zu 349,5 g (3,0 mol) Chlorsulfonsäure und 2 g Amidosulfonsäure werden 135,2 g (1,0 mol) Acetanilid portionsweise so zugegeben, daß die Temperatur bei 40°C bleibt. Anschließend wird auf 60°C geheizt und 60 Minuten bei dieser Temperatur nachgerührt, dann tropft man 142,8 g (1,2 mol) Thionylchlorid über 2 Stunden zu und rührt bis zum Ende der Gasentwicklung nach. Zur Aufarbeitung wird in Eiswasser getropft, die ausgefallenen Kristalle abgesaugt und mit Wasser gewaschen. Es werden 394,9 g 4-Acetamidobenzolsulfonsäurechlorid (Wassergehalt: 41,7 %; Chloridgehalt: 58,1 %) entsprechend 230,2 g (98,5 %) trocken gerechnet mit einem Schemlzpunkt von 139°C erhalten.

### Beispiel 15 (4-Acetamidobenzosulfonsäurechlorid) - Vergleichsbeispiel -

135,2 g (1,0 mol) Acetanilid, 349,5 g (3,0 mol) Chlorsulfonsäure und 142,8 g (1,2 mol) Thionylchlorid werden analog Beispiel 14, jedoch ohne Amidosulfonsäure, umgesetzt. Es werden 358,5 g 4-Acetaminobenzolsulfonsäurechlorid (Wassergehalt: 39,4 %, Chloridgehalt: 60,2 %), entsprechend 217,3 g (93,0 % d.Th.), trocken gerechnet, mit einem Schmelzpunkt von 139°C erhalten.

### Beispiel 16 (3-Chlorsulfonylbenzoesäure)

In eine Mischung aus 349,5 g (3,0 mol) Chlorsulfonsäure, 20 g 96 %iger Schwefelsäure und 1 g Amidosulfonsäure werden 122,1 g (1,0 mol) Benzoesäure eingetragen. Die so erhaltene Reaktionsmischung wird innerhalb von 3 Stunden auf 120°C geheizt und bis zum Ende der Gasentwicklung nachgerührt. Anschließend wird auf 70°C abgekühlt und über 2 Stunden 119,0 g (1,0 mol) Thionylchlorid zugetropft. Nachdem 30 Minuten bei 80°C nachgerührt wurde, wird die Reaktionsmischung bei 10°C in Eiswasser eingetropft, die ausgefallenen Kristalle abgesaugt und mit Eiswasser gewaschen. Es werden 232,1 g 3-Chlorsulfonylbenzoesäure (Wassergehalt: 10 %), entsprechend 208,9 g (94,7 % d.Th.), trocken gerechnet, mit einem Schmelzpunkt von 129 - 131°C erhalten.

### Beispiel 17 (3-Chlorsulfonylbenzoesäure) - Vergleichsbeispiel -

122,1 g (1,0 mol) Benzoesäure, 349,5 g (3,0 mol) Chlorsulfonsäure, 20 g 96 %ige Schwefelsäure und 119 g (1,0 mol) Thionylchlorid werden analog Beispiel 16, jedoch ohne Zusatz von Amidosulfonsäure, umgesetzt. Es werden 198,3 g 3-Chlorsulfonylbenzoesäure (Wassergehalt: 8,2 %), entsprechend 182,0 g (82,5 % d.Th.), trocken gerechnet, erhalten.

### Beispiel 18 (6-Benzoxazolsulfonsäurechlorid)

Zu 349,5 g (3,0 mol) Chlorsulfonsäure und 1 g Amidosulfonsäure werden 135 g (1,0 mol) Benzoxazolon so eingetragen, daß die Temperatur nicht über 40°C steigt. Anschließend wird auf 60°C erhitzt und 1 Stunde bei dieser Temperatur nachgerührt. Dann tropft man 142,8 g (1,2 mol) Thionylchlorid zu und rührt bis zum Ende der Gasentwicklung nach. Die Reaktionsmischung wird in Eiswasser getropft, die ausgefallenen Kristalle abgesaugt und mit Wasser gewaschen. Es fallen 264,2 g feuchtes 6-Benzoxazolsulfonsäurechlorid mit einem Wassergehalt von 21,2 % und einem Schmelzpunkt von 188 - 190°C, entsprechend einer Ausbeute von 208,5 g (89,3 % d.Th.), an.

### Beispiel 19 (6-Benzoxazolonsulfonsäurechlorid) - Vergleichsbeispiel -

349,5 g (3,0 mol) Chlorsulfonsäure, 135 g (1,0 mol) Benzoxazolon und 142,8 g (1,2 mol) Thionylchlorid werden, wie in Beispiel 18 beschreiben, jedoch ohne Zusatz von Amidosulfonsäure, umgesetzt.

Es fallen 231,7 g 6-Benzoxazolonsulfonsäurechlorid mit einem Wassergehalt von 19,4 %, entsprechend einer Ausbeute von 186,8 g (80,0 % d.Th.), an.

### Beispiel 20 (3-Nitrobenzolsulfonsäurechlorid)

Zu 582,5 g (5,0 mol) Chlorsulfonsäure und 1 g Amidosulfonsäure läßt man 123,1 9 (1,0 mol) Nitrobenzol bei Raumtemperatur schnell zulaufen. Anschließend wird auf 105°C geheizt und 6 Stunden bei dieser Temperatur nachgerührt. Zur Aufarbeitung wird die Reaktionsmischung in Eiswasser getropft. Die ausgefallenen Kristalle werden abgesaugt und mit Wasser gewaschen. Es fallen 194,6 g trockenes 3-Nitrobenzolsulfonsäurechlorid mit einem Schmelzpunkt von 59 - 61°C, entsprechend einer Ausbeute von 87,8 % d.Th., an.

### Beispiel 21 (3-Nitrobenzolsulfonsäurechlorid) - Vergleichsbeispiel -

123,1 g (1,0 mol) Nitrobenzol und 582,5 g (5,0 mol) Chlorsulfonsäure werden, wie in Beispiel 20 beschrieben, jedoch ohne Zusatz von Amidosulfonsäure, umgesetzt. Es fallen 175,1 g trockenes 3-Nitrobenzolsulfonsäurechlorid, entsprechend einer Ausbeute von 79,0 % d.Th., an.

### Beispiel 22 (2-Nitrochlorbenzol-4-sulfonsäurechlorid)

157,6 g (1,0 mol) 2-Chlornitrobenzol werden zu 699 g (6,0 mol) Chlorsulfonsäure und 2 g Amidosulfonsäure so zugegeben, daß die Temperatur 40°C nicht überschreitet. Nach beendeter Zugabe wird auf 100°C geheizt und 6 Stunden bei dieser Temperatur nachgerührt. Die Reaktionsmischung wird bei 15°C in Wasser getropft, die ausgefallenen Kristalle abgesaugt und mit Wasser nachgewaschen. Es fallen 236,2 g 2-Nitrochlorbenzol-4-sulfonsäurechlorid mit einem Wassergehalt von 1,5 %, entsprechend 232,7 g 2-Nitrochlorbenzol-4-sulfonsäurechlorid (90,8 % d.Th.), an.

### Beispiel 23 (2-Nitrochlorbenzol-4-sulfonsäurechlorid) - Vergleichsbeispiel -

157,6 g (1,0 mol) 2-Chlornitrobenzol und 699 g (6,0 mol) Chlorsulfonsäure werden gemäß Beispiel 22, jedoch ohne Zusatz von Amidosulfonsäure, umgesetzt. Es fallen 212,8 g 2-Nitrochlorbenzol-4-sulfonsäurechlorid mit einem Wassergehalt von 1,2 %, entsprechend 210,2 g 2-Nitrochlorbenzol-4-sulfonsäurechlorid (82,1 % d.Th.), an.

### Beispiel 24 (Naphthalin-1,5-disulfonsäurechlorid)

64,1 g (0.5 mol) Naphthalin werden unter Eiskühlung in 640,7 g (5.5 mol) Chlorsulfonsäure, die mit 1 g Amidosulfonsäure versetzt ist, eingetragen. Anschließend wird 4 Stunden bei Raumtemperatur nachgerührt. Die Reaktionsmischung wird in Eiswasser eingetropft, die ausgefallenen Kristalle abgesaugt und mit Wasser nachgewaschen. Es fallen 172,1 g Naphthalin-1,5-disulfonsäurechlorid mit einem Wassergehalt von 26,5 % und einem Chloridgehalt von 71,6 %, entsprechend 123,2 g (75,7 %), an.

### Beispiel 25 (Naphthalin-1,5-disulfonsäurechlorid) - Vergleichsbeispiel -

64,1 g (0.5 mol) Naphthalin und 640,7 g (5,5 mol) Chlorsulfonsäure werden gemäß Beispiel 26, jedoch ohne Zusatz von Amidosulfonsäure, umgesetzt. Es fallen 139,4 g Naphthalin-1,5-disulfonsäurechlorid mit einem Wassergehalt von 25,8 % und einem Chloridgehalt von 72,5 %, entsprechend 101,1 g (62,2 %), an.

## Patentansprüche

1. Verfahren zur Herstellung aromatischer Sulfonsäurechloride der allgemeinen Formel I 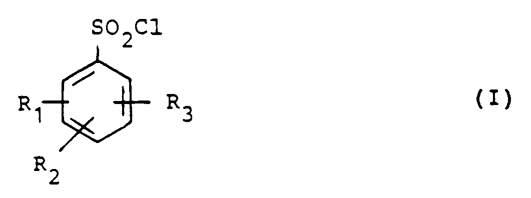 in welcher R₁, R₂ und R₃ gleich oder verschieden sind und Wasserstoff-, Fluor-, Chlor-, Brom- oder Jodatome, (Alkyl(C₁-C₄)-, Acetylamino-, Nitro- oder Carboxylgruppen bedeuten, oder R₁ und R₂ zusammen einen aromatischen oder heteroaromatischen Ring mit 5 oder 6 Ringgliedern bilden, welcher durch Fluor-, Chlor-, Brom-oder Jodatome, Alkyl(C₁-C₄)-, Acetylamino-, Nitro- oder Carboxylgruppen substituiert sein kann, durch Umsetzung von Aromaten der allgemeinen Formel II 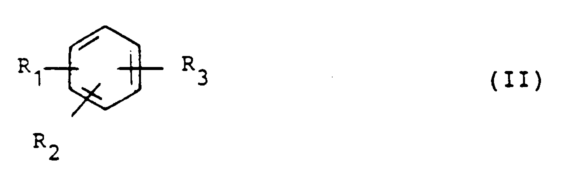 in welcher R₁, R₂ und R₃ die vorstehend genannten Bedeutungen haben, mit Chlorsulfonsäure im Überschuß oder mit Chlorsulfonsäure bzw. Oleum und Thionylchlorid, dadurch gekennzeichnet, daß man in Gegenwart von Amidosulfonsäure als Katalysator umsetzt.

2. Verfahren nach Anspruch 1, dadurch gekennzeichnet, daß man in Gegenwart von etwa 0,1 bis etwa 20 Gewichtsprozent Amidosulfonsäure, bezogen auf den Aromat der genannten allgemeinen Formel II, umsetzt.

3. Verfahren nach mindestens einem der Ansprüche 1 und 2, dadurch gekennzeichnet, daß man in Gegenwart von etwa 0,5 bis etwa 5 Gewichtsprozent Amidosulfonsäure, bezogen auf den Aromat der genannten allgemeinen Formel II, umsetzt.

4. Verfahren nach mindestens einem der Ansprüche 1 bis 3, dadurch gekennzeichnet, daß man bei Normal- oder Überdruck arbeitet.

5. Verfahren nach mindestens einem der Ansprüche 1 bis 4, dadurch gekennzeichnet, daß die Amidosulfonsäure der Chlorsulfonsäure zu Beginn der Umsetzung vollständig zugesetzt wird.

6. Verfahren nach mindestens einem der Ansprüche 1 bis 4, dadurch gekennzeichnet, daß die Amidosulfonsäure der Chlorsulfonsäure zu Beginn der Umsetzung nur teilweise zugesetzt wird und während der Umsetzung weitere Amidosulfonsäure zudosiert wird.

7. Verfahren nach mindestens einem der Ansprüche 1 bis 4, dadurch gekennzeichnet, daß man bei Verwendung von Chlorsulfonsäure oder Oleum als Sulfonierungsmittel die Amidosulfonsäure dem nach Umsetzung des Aromaten der genannten allgemeinen Formel II mit dem genannten Sulfonierungsmittel angefallenen Reaktionsgemisch zugibt und anschließend mit Thionylchlorid zum Arylsulfochlorid umsetzt.

## Claims

1. A process for the preparation of aromatic sulfonyl chlorides of the formula I 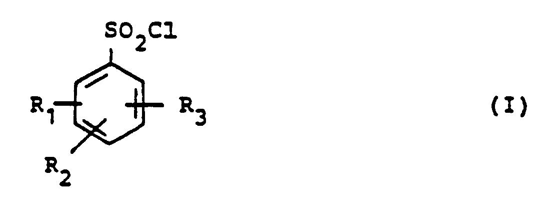 in which R₁, R₂ and R₃ are identical or different and are hydrogen, fluorine, chlorine, bromine or iodine atoms, alkyl(C₁-C₄), acetamido, nitro or carboxyl groups, or R₁ and R₂ together form an aromatic or heteroaromatic ring having 5 or 6 ring members, which can be substituted by fluorine, chlorine, bromine or iodine atoms, alkyl(C₁-C₄), acetamido, nitro or carboxyl groups, by reaction of aromatic compounds of the formula II 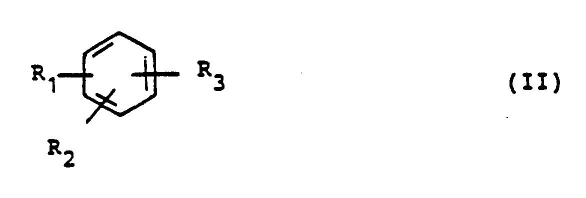 in which R₁, R₂ and R₃ have the abovementioned meanings, with chlorosulfonic acid in excess or with chlorosulfonic acid or oleum and thionyl chloride, which comprises reacting in the presence of sulfamic acid as a catalyst.

2. The process as claimed in claim 1, which comprises reacting in the presence of about 0.1 to about 20 percent by weight of sulfamic acid, relative to the aromatic compound of said formula II.

3. The process as claimed in at least one of claims 1 and 2, which comprises reacting in the presence of about 0.5 to about 5 percent by weight of sulfamic acid, relative to the aromatic compound of said formula II.

4. The process as claimed in at least one of claims 1 to 3, which process is carried out at normal or elevated pressure.

5. The process as claimed in at least one of claims 1 to 4, wherein the sulfamic acid is completely added to the chlorosulfonic acid at the start of the reaction.

6. The process as claimed in at least one of claims 1 to 4, wherein the sulfamic acid is only partially added to the chlorosulfonic acid at the start of the reaction and further sulfamic acid is metered in during the reaction.

7. The process as claimed in at least one of claims 1 to 4, wherein, when using chlorosulfonic acid or oleum as the sulfonating agent, the sulfamic acid is added to the reaction mixture obtained after reaction of the aromatic compound of said formula II with said sulfonating agent and the mixture is then reacted with thionyl chloride to give arylsulfonyl chloride.

## Revendications

1. Procédé pour la préparation de chlorures d'acides sulfoniques aromatiques de formule générale I 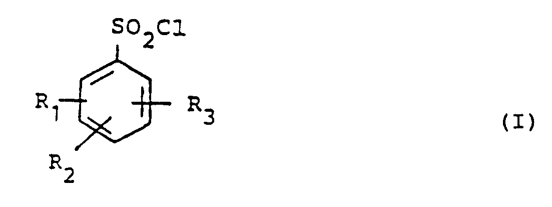 dans laquelle R₁, R₂ et R₃ sont identiques ou différents et représentent des atomes d'hydrogène, de fluor, de chlore, de brome ou d'iode, des groupes alkyle (C₁-C₄), acétylamino, nitro ou carboxyle, ou bien où R₁ et R₂ constituent ensemble un noyau aromatique ou hétéroaromatique avec 5 ou 6 chaînons, qui peut être substitué par des atomes de fluor, de chlore, de brome ou d'iode, des groupes alkyle (C₁-C₄), acétylamino, nitro ou carboxyle, par réaction de composés aromatiques de formule générale II 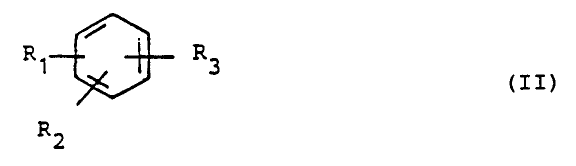 dans laquelle R₁, R₂ et R₃ ont la signification ci-dessus, avec de l'acide chlorosulfonique en excès ou de l'acide chlorosulfonique ou respectivement un oléum et avec du chlorure de thionyle, caractérisé en ce que la réaction s'effectue en présence d'acide amidosulfonique comme catalyseur.

2. Procédé selon la revendication 1, caractérisé en ce que la réaction s'effectue en présence d'environ 0,1 à environ 20 pour cent en poids d'acide amidosulfonique, par rapport au composé aromatique de formule générale II citée.

3. Procédé selon l'une au moins des revendications 1 et 2, caractérisé en ce que la reaction s'effectue en présence d'environ 0,5 à environ 5 pour cent en poids d'acide amidosulfonique, par rapport au composé aromatique de formule générale II citée.

4. Procédé selon l'une au moins des revendications 1 à 3, caractérisé en ce que l'on opère à la pression normale ou en surpression.

5. Procédé selon l'une au moins des revendications 1 à 4, caractérisé en ce que l'acide amidosulfonique est ajouté complètement à l'acide chlorosulfonique au début de la réaction.

6. Procédé selon l'une au moins des revendications 1 à 4, caractérisé en ce que l'acide amidosulfonique n'est ajouté que partiellement à l'acide chlorosulfonique au début de la réaction et que l'on ajoute encore de manière dosée de l'acide amidosulfonique au cours de la réaction.

7. Procédé selon l'une au moins des revendications 1 à 4, caractérisé en ce que, si l'on utilise de l'acide chlorosulfonique ou un oléum comme agent de sulfonation, l'on ajoute l'acide amidosulfonique au mélange de réaction qui se forme à l'issue de la réaction du composé aromatique de formule générale II avec l'agent de sulfonation précédemment évoqué et on fait réagir ensuite avec du chlorure de thionyle pour former le sulfochlorure d'aryle.
